# EUROPEAN PATENT APPLICATION

(11) **EP 2 689 836 A1**
(43) Date of publication of application: **29.01.2014**
(21) Application number: 12178056.3
(22) Date of filing: 26.07.2012
(51) Int. Cl.: B01J 13/18, A23L 1/22, A61K 8/11, A61K 9/50, C11D 3/50

(54) **Composition of microcapsules with a silica shell and a method for preparing them**

(71) Applicant: BASF SE, 67056 Ludwigshafen (DE)
(72) Inventor: The designation of the inventor has not yet been filed
(74) Representative: Reitstötter - Kinzebach

(57) **Abstract**

A process for the preparation of a microcapsule composition, wherein the shell of the microcapsules is essentially made of silica and the core comprises at least one lipophilic component, comprising the steps of:
a) providing an aqueous dispersion comprising at least one lipophilic component (A) and
b1) adding to the aqueous dispersion provided in step a) a water glass solution and an acid, or
b2) adding to the aqueous dispersion provided in step a) a silicic acid solution and a base,

wherein the addition is effected such that the pH of the mixture resulting during the addition of the water glass solution in step b1) or during the addition of silicic acid solution in step b2) is kept in a range of 6 to 9.

## Description

The present invention essentially relates to a microcapsule composition as core-shell particles and a method of making them. The microcapsule composition comprises a core of a lipophilic liquid or viscous substance surrounded by a shell of amorphous silica of various thicknesses. The method is based on emulsification of the liquid or viscous substance in water using different emulsifying agents and forming the shell by a sol-gel process in which the condensation of the silica precursor is done by simultaneously adding the silica precursor (metal-silicates or silicic acid) and an acid or base to the emulsion and thus keeping the pH constant during addition in a pH range of 6 to 9.

Polymer additives like flame retardants, UV-stabilizers, UV-protecting agents etc. can occur in solid, liquid or viscous form. Especially for technical thermoplastic polymers, liquid and viscous forms at room temperature or at temperatures at which the compounding of the polymers is performed are a problem since these liquid or viscous additives cannot sufficiently be incorporated (compounded) with the polymer matrix. Phase separation and leaching of the additives are the main problems during the compounding and storing of the material.

Thus, several techniques are used to make a solid material/powder of these additives. These techniques involve mixing of amorphous silica particles with the active ingredients, encapsulation of the actives with certain polymer shells or other polymeric (mainly organic) shell materials (see e.g. Angew. Chemie 1975, 87, 556). This invention, however, essentially relates to encapsulation of those additives with a silica shell by a sol-gel process forming microcapsules with a liquid or viscous core and a silica shell.

Microcapsules composed of a shell prepared by a sol-gel process have been described in various publications.

US 6,303,149, US 6,238,650, US 6,468,509 and US 6,436,375, US 7,923,030, US 2002/0037261, US 20050037087, US 20020064541, US 2004/0256748 and WO 00/09652, WO 2008/072239, WO 00/72806, WO 01/80823, WO 03/039510, WO 00/71084, WO 2005/009604, WO 2004/081222 disclose sol-gel microcapsules and their preparation.

EP 0934773, US 6,337,089 and US 6,251,313 describe microcapsules having a core material and a wall or shell made of organopolysiloxanes.

A method for forming microcapsules and micromatrix bodies having an interior liquid and water-insoluble phase containing an active, water immiscible ingredient and an organopolysiloxanes forming the shell is described in US 4,931,362.

US 7,758,888 discloses core-shell microcapsules with a shell material made of a metal oxid inorganic polymer, whereas microcapsules prepared by a sol-gel process are also disclosed in US 6,855,335 and WO 03/066209.

However, all of the inventions describe microcapsules prepared by a sol-gel process with a sol-gel precursor consisting of an organosilicon compound or a metal alkoxide, semi-metal alkoxide, or a metal ester (monomers and partly condensed polymers). Usually, the process involves emulsifying the sol-gel precursor and the active ingredient in a water phase and upon pH-adjustment the silica shell or matrix body is formed.

WO 2007/129849 discloses a method for preparing mesoporous silica, wherein water glass (sodium silicate) is added to a mixture of surfactant and HCl and wherein simultaneously the pH value is adjusted in a range from 5 to 7 at a temperature of 30 to 50°C. The resulted mesoporous silica can be used as heat isolator or absorbents. This document does not teach process conditions that lead to the formulation of microcapsules, wherein the shell is essentially made of silica.

Alkali metal silicate solution as silica precursor is also disclosed in US 6,132,773 and EP 0897414 to encapsulate a support of CaC0₃ with a silica shell. Additionally, EP 0143221 discloses the use of amorphous silica particles to encapsulate volatile organic liquids.

Such microcapsules are used in various applications, where the active ingredient should be protected from the environment, e.g. colorants in cosmetics, food colors, sunscreen compositions or other applications, where delivery of the active is of benefit (e.g. topical delivery onto the skin or controlled release properties in medical applications). However, none of theses microcapsules are used to incorporate liquid or viscous substances like polymer additives and then be used themselves as additives for polymer matrices so far.

It is an object of the present invention to provide a process for preparing a microcapsule composition, wherein the shell is made of silica and the core comprises at least one lipophilic substance like polymer additives. Further, it is an object of the present invention to provide a microcapsule composition, wherein the shell is made of silica and the core comprises at least one lipophilic substance like polymer additives which can sufficiently be incorporated in a polymer matrix without separation and leaching of the polymer additives.

Surprisingly, these objects could be achieved by preparation of a microcapsule composition, wherein the shell of the microcapsules is made of silica by an interfacial sol-gel-process using water glass or silicic acid as silica source and the core comprises at least one lipophilic substance.

Therefore, the present invention relates to a process for the preparation of a microcapsule composition, wherein the shell of the microcapsules is essentially made of silica and the core comprises at least one lipophilic component, comprising the steps of:
a) providing an aqueous dispersion comprising at least one lipophilic component (A), and
b1) adding to the aqueous dispersion provided in step a) a water glass solution and an acid, or
b2) adding to the aqueous dispersion provided in step a) a silicic acid solution and a base,
wherein the addition is effected such that the pH of the mixture resulting during the addition of the water glass solution in step b1) or during the addition of silicic acid solution in step b2) is kept in a range of 6 to 9.

The process according to the invention allows the feasible and effective preparation of a microcapsule composition, wherein the shell of the microcapsules is essentially made of silica and the core comprises at least one lipophilic component. Furthermore, a great variety of different lipophilic components (e.g. active ingredients, like polymer additives, or components that modify/add to the physical material properties, like latent heat storage materials) can be encapsulated with a thermally and mechanically stable shell. This stable form can be incorporated into or compounded with a great number of different carrier materials, e.g. a polymer matrix or a hydraulically setting mass. Moreover, by utilizing water glass as a silica containing precursor, a cheap starting material is used that allows a cost-effective production.

One of the objects of the present invention was to synthesize a material consisting of an encapsulated liquid or viscous substances like polymer additive (like flame retardant, UV-absorber, UV stabilizer, etc.) as a core-shell particle with a shell made of silica and the active being the core. Furthermore, these microcapsule compositions were used as polymer additives themselves, meaning they were incorporated (compounded) into a polymer matrix to show better process ability and workability (performance) than the additive in its pure (non-encapsulated) form or as a physical mixture of the additive and an amorphous silica support.

In the context of the present invention, a shell of the microcapsules essentially made of silica means that at least 90% by weight, preferably at least 95% by weight, in particular at least 99% by weight, of the shell material is silica (SiO₂). The shell may contain minor amounts of further covalently bound atoms, in particular C and/or P.

### Step a)

In step a) an aqueous dispersion comprising at least one lipophilic component (A) and optionally at least one surfactant is provided.

In a preferred embodiment of the process according to the invention, the lipophilic component (A) is liquid at least under the reaction conditions in step b1) or b2). In the context of the invention the term "liquid" is understood in a broad sense and means a material that is flowable under the conditions of the process of the invention. Preferably, this viscosity of the lipophilic compound is in a range of from 1 to 10⁵ mPas, determined as Brookfield viscosity at 20°C with spindle. In a preferred embodiment of the process according to the invention, the lipophilic component is liquid at least at 23°C and 1013 mbar.

In the sense of the invention, the term "lipophilic component" is understood in a broad sense. It encompasses a single lipophilic compound, a mixture comprising at least two lipophilic compounds and a solution of at least one compound in a lipophilic solvent.

The lipophilic component (A) is liquid or viscous at the time it is dispersed and usually it is liquid or viscous at ambient temperature. It can be an undiluted lipophilic component or it can be a solution of the lipophilic component in a lipophilic solvent or a dispersion of a lipophilic component in a suitable dispersant. If the lipophilic component A is solubilised in a solvent, the solvent is preferably selected from aliphatic and aromatic hydrocarbons, e. g. toluene, decane, hexane. If the lipophilic component A is dispersed in a dispersant, the dispersant is preferably a hydrophilic solvent, in particular water.

The lipophilic components (A) are in general components which have only limited solubility in water. The solubility of the lipophilic component (A) in water at 23°C and 1013 mbar is preferably ≤ 50 mg/mL, more preferably ≤ 5 mg/mL, in particular ≤ 1 mg/mL.

Lipophilic components (A) that are used can be various organic substances. Suitable lipophilic components are e.g. selected from lipophilic flame retardants, lipophilic UV-stabilizers, lipophilic UV-protecting agents, lipophilic fragrances, lipophilic flavours, lipophilic vitamins, lipophilic aromatic compounds, lipophilic cosmetic or therapeutic agents, etc.

Preferably, the lipophilic component (A) is selected from hydrocarbons, waxes, oils, fatty acids, fatty amines, esters, dibasic acids, 1-halides, alcohols, aromatic compounds, clathrates, polymers, adhesives, flavours and perfume oils and mixtures thereof.

Suitable aliphatic hydrocarbon compounds are straight-chain alkanes or paraffinic hydrocarbons, branched-chain alkanes, unsaturated hydrocarbons, halogenated hydrocarbons, and alicyclic hydrocarbons such as hexane, cyclohexane, decane, chloroparaffines, fluorinated hydrocarbons, saturated or unsaturated C₁-C₄₀-hadrocarbons which are branched or linear, e. g. n-tetradecane, n-pentadecane, n-hexadecane, n-heptadecane, n-octadecane, n-nonadecane, n-eicosane, n-heneicosane, n-docosane, n-tricosane, n-tetracosane, n-pentacosane, n-hexacosane, n-heptacosane, n-octacosane, also cyclic hydrocarbons, e. g. cyclohexane, cyclodecane; halogenated hydrocarbons such as chloroparaffines, bromooctadecane, bromopentadecane, bromononadecane, bromeicosane, bromodocosane;
Suitable aromatic compounds are benzene, naphthalene, alkylnaphthalenes, biphenyl, o- or n-terphenyl, xylene, toluene dodecylbenzene, C₁-C₄₀-alkyl-substituted aromatic hydrocarbons, such as dodecylbenzene, tetradecylbenzene, hexadecylbenzene, hexylnaphtalene or decylnaphtalene;
Suitable saturated or unsaturated C₆-C₃₀-fatty acids are lauric acid, stearic acid, oleic acid or behenic acid, preferably eutectic mixtures of decanonic acid with for example myristic, palmitic or lauric acid;
Suitable alcohols are primary, secondary alcohols, tertiary alcohols which are not soluble in water, dipentaerythritol, pentaglycerine, neopentyl glycol, tetramethylol propane, fatty alcohols, such as lauryl, stearyl, oleyl, myristryl, cetyl alcohol, mixtures, such as coconut fatty alcohol and also oxo alcohols which are obtained by hydroformylation of α-olefins and further reactions;
Suitable fatty amines are C₆-C₃₀-fatty amines, such as decylamine, dodecylamine, tertadecylamine or hexadecylamine;
Suitable esters are esters of carboxylic acids, esters of sulfonic acid, esters of sulphuric acid or esters of phosphorous acid. Suitable esters of carboxylic acids are e.g. C₁-C₁₀-alkyl esters of fatty acids, mono and diesters of dicarboxylic acids, alkylene carbonates, etc. Preferred esters of carboxylic acids are the methyl esters and ethyl esters of fatty acids, ethylene carbonate, C₁-C₁₀-alkyl esters of C₁-C₆- dicarboxylic acids. Especially preferred are propyl palmitare, methyl stearate or methyl palmitate, their eutectic mixtures, or methyl cinnamate;
Suitable waxes are natural and synthetic waxes such as montan waxes, montan ester waxes, carnauba waxes, polyethylene wax, oxidized waxes, polyvinyl ether wax, ethylene-vinyl acetate wax or hard waxes obtained from Fischer-Tropsch process;
Suitable oils are petroleum spirit, mineral oil or natural oils;
Suitable clathrates are semi-clathrates or gas clathrates;
A suitable anhydride is stearic anhydride;
Suitable polymers are polyethylene, polypropylene, polypropylene glycol, polytetramethylene glycol, polypropylene malonate, polyneopentyl glycol sebacate, polypentane glutarate, polyvinyl myristate, polyvinyl stearate, polyvinyl laurate, polyhexadecyl methacrylate, polyoctadecyl methacrylate, polyesters produced by polycondensation of glycols (or their derivatives) with diacids (or their derivatives), and copolymers, such as polyacrylate or poly(meth)acrylate with alkyl hydrocarbon side chain or with polyethylene glycol side chain and copolymers including polyethylene, polypropylene, polypropylene glycol, or polytetramethylene glycol;
and mixtures of these substances.

These liquids can further comprise active compounds, such as crop protection agents or pharmaceutical dyes or color formers in dissolved or suspended form.

In a preferred embodiment, the lipophilic component (A) comprises or consists of a flame retardant. Flame retardant compounds which are used in the invention can, for example, be organic flame retardants, such as halogen-containing flame retardants, nitrogen-based flame retardants, aromatic or aliphatic esters of the phosphoric acid or mixtures thereof.

Suitable halogen-containing flame retardants are e.g. brominated flame retardants, preferred polybrominated bisdiphenylethers, polybrominated biphenyls or further brominated hydrocarbons. Preferred brominated flame retardants are pentabromodiphenyl ether (PentaBDE), octabromodiphenyl ether (OctaBDE) and decabromodiphenyl ether (DecaBDE), tetrabromobisphenol A (TBBPA), hexabromocyclododecane (HBCD) and mixtures thereof.

Suitable halogen-containing flame retardants are also chlorinated flame retardants, in particularchlorendic acid (1,4,5,6,7,7-hexachlorobicyclo[2.2.1]-hept-5-ene-2,3-dicarboxylic acid) and chlorinated paraffins.

Suitable nitrogen-based flame retardants are melamine and urea and mixtures thereof.

Suitable organophosphorus flame-retardants are e.g. aromatic and aliphatic organophosphates, organophosphonates, organophosphinates or organophosphorus compounds containing at least one halogen atom. Preferred organophosphorus flame-retardants are tris(2-chloroethyl) phosphate (TCEP), tris(1-chloro-2-propyl) phosphate (TCPP), tris(1,3-dichloro-2-propyl) phosphate (TDCPP), triphenyl phosphate (TPP), triethyl phosphate, isodecyl diphenyl phosphate, tris-(2-ethylhexyl) phosphate (TEHP), tri-n-butyl phosphate, tri-isobutyl phosphate, tricresyl phosphate (TCP), isopropylated triphenyl phosphate (ITP) with different isopropyl rates (e.g. mono-, bis- and tris-isopropyl phenyl phosphate), resorcinol-bis(diphenyl phosphate) (RDP), bisphenol-A-bis(diphenyl phosphate) (BDP) and mixtures thereof.

The flame retardant compounds can perform their function as soon as they are released from the microcapsules. The shell made of silica has no effect on the action of the flame retardant compounds and in particular has no negative impact on the efficiency of the flame retardant compounds.

The lipophilic component (A) can, for example, be an UV-absorber. UV-absorber compounds which are used in the invention can, for example, be used in coatings, paints, plastic materials, sealants, cremes.

The UV-absorber compounds can perform their function while they are encapsulated or as soon as they are decapsulated. The shell made of silica has no effect on the action of the UV-absorber compounds and has no negative impact on the efficiency of the UV-absorber compounds.

The lipophilic components can be, for example, a UV stabilizer. UV stabilizer compounds which are used in the invention can, for example, be used in coatings, paints, plastic materials, sealants, pigments.

The UV stabilizer compounds can perform their function while they are encapsulated or as soon as they are decapsulated. The shell made of silica has no effect on the action of the UV stabilizer compounds and has no negative impact on the efficiency of the UV stabilizer compounds.

In a further embodiment, the lipophilic component (A) comprises or consists of at least one fragrance. Suitable lipophilic fragrances employed according the present invention are conventional ones known in the art. Suitable perfume compounds and compositions can be found in the art including U.S. Pat. Nos. 4,145,184, Brain and Cummins, issued Mar. 20, 1979; 4,209,417, Whyte, issued Jun. 24, 1980; 4,515,705, Moeddel, issued May 7, 1985; 4,152,272, Young, issued May 1, 1979; 5,378,468 Suffis et al.; U.S. Pat. No. 5,081,000 Akimoto et al., issued Jan. 14, 1992; U.S. Pat. No. 4,994,266 Wells, issued Feb. 19, 1991; U.S. Pat. No. 4,524,018 Yemoto et al., issued Jun. 18, 1985; U.S. Pat. No. 3,849,326 Jaggers et al., issued Nov. 19, 1974; U.S. Pat. No. 3,779,932 Jaggers et al., issued Dec. 18, 1973; JP 07-179,328 published Jul. 18, 1995; JP 05-230496 published Sep. 7, 1993; WO 96/38528 published Dec. 5, 1996; WO 96/14827 published May 23, 1996; WO 95/04809 published Feb. 16, 1995; and WO 95/16660 published Jun. 22, 1995; all of said U.S. patents and U.S. references being incorporated herein by reference. In addition, P. M. Muller, D. Lamparsky Perfumes Art, Science, & Technology Blackie Academic & Professional, (New York, 1994) is included herein by reference.

Fragrances can be classified according to their volatility. The highly volatile, low boiling, perfume ingredients typically have boiling points of about 250°C or lower. The moderately volatile perfume ingredients are those having boiling of from about 250°C to about 300°C. The less volatile, high boiling, perfume ingredients are those having boiling points of about 300°C or higher. Many of the perfume ingredients as discussed hereinafter along with their odor and/or flavor characters, and their physical and chemical properties, such as boiling point and molecular weight, are given in "Perfume and Flavor Chemicals (Aroma Chemicals)," Steffen Arctander, published by the author, 1969, incorporated herein by reference.

Examples of highly volatile, low boiling, perfume ingredients are: anethole, benzaldehyde, benzyl acetate, benzyl alcohol, benzyl formate, iso-bornyl acetate, camphene, cis-citral (neral), citronellal, citronellol, citronellyl acetate, paracymene decanal, dihydrolinalool, dihydromyrcenol, dimethyl phenyl carbinol, eucalyptol, geranial, geraniol, geranyl acetate, geranyl nitrile, cis-3-hexenyl acetate, hydroxycitronellal, d-limonene, Inalool, linalool oxide, linalyl acetate, linalyl propionate, methyl anthranilate, alpha-methyl ionone, methyl nonyl acetaldehyde, methyl phenyl carbonyl acetate, laevo-menthyl acetate, menthone, iso-menthone, myrcene, lyrcenyl acetate, myrcenol, mero, meryl acetate, nonyl acetate, phenyl ethyl alcohol, alpha-pinene, beta-pinene, gamma phenene, alpha-terpineol, beta-terpineol, terpinyl acetate, and vertenex (para-tertiary-butyl cyclohexyl acetate). For example, lavadin contains as major components: linalool; linalyl acetate; geraniol; and citronellol. Lemon oil and orange terpenes both contain about 95% of d-limonene.

Examples of moderately volatile perfume ingredients are: amyl cinnamic aldehyde, isoamyl salicylate, beta-caryophyllene, cedrene, cinnamic alcohol, coumarine, dimethyl benzyl carbonyl acetate, ethyl vanillin, eugenol, iso-eugenol, heliotropine, 3-cis-hexenyl salicylate, hexyl salicylate, lilial (para-tertiarybutyl-alpha-methyl hydrocinnamic aldehyde), gamma-methyl ionone, merolidol, patchouli alcohol, phenyl hexanol, geta-selinene, trichloromethyl phenyl carbonyl acetate, triethyl citrate, vanillin, and veratrum aldehyde. Cedar terpenes are composed mainly of alpha-cedrene, beta-cedrene, and other C₁₅H₂₄ sesquiterpenes.

Examples of the less volatile, high boiling, perfume ingredients are: benzophenone, benzyl salicylate, ethylene brassylate, galaxolide (1,3,4,6,7,8-hexahydro-4,6,6,7,8,8-hexamethyl-cyclopenta-gamma-2-benzopyran), hexyl cinnamic aldehyde, lyral (4-(4-hydroxy-4-methyl pentyl)-3-cyclohexene-10-caroxaldehyde), methyl cedrylone, methyl dihydro jasmonate, methyl-beta-naphthyl ketone, musk indanone, musk ketone, musk tibetene, and phenylethyl phenyl acetate.

The fragrances compounds can perform their function as soon as they are released. The shell made of silica has no effect on the action of the fragrances compounds and has no negative impact on the efficiency of the fragrances compounds.

The lipophilic components can be, for example, water insoluble liquid chemicals which can protect the lipophilic component, for example during storage or transport.

The lipophilic components can be, for example, pharmaceuticals or sensitive chemical materials. Radioactively marked material can be encapsulated for cancer treatment.

In a preferred embodiment the lipophilic component is dispersed in an aqueous medium with the aid of a surfactant. The surfactant is an anionic, cationic, amphoteric, non ionic surfactant or a mixture thereof, preferably non-ionic surfactants.

The anionic surfactants include, for example, carboxylates, in particular alkali metal, and ammonium salts of fatty acids, e.g. potassium stearate, which are usually also referred to as soaps; acyl glutamates; sarcosinates, e.g. sodium lauroyl sarcosinate; taurates; methylcelluloses; alkyl phosphates, in particular mono- and diphosphoric acid alkyl esters; sulfates, in particular alkyl sulfates and alkyl ether sulfates; sulfonates, further alkyl- and alkylarylsulfonates, in particular alkali metal and ammonium salts of arylsulfonic acids, and also alkyl-substituted arylsulfonic acids, alkylbenzenesulfonic acids, such as, for example, ligno- and phenolsulfonic acid, naphthalene- and dibutylnaphthalenesulfonic acids, or dodecylbenzenesulfonates, alkylnaphthalenesulfonates, alkyl methyl ester sulfonates, condensation products of sulfonated naphthalene and derivatives thereof with formaldehyde, condensation products of naphthalenesulfonic acids, phenolic and/or phenolsulfonic acids with formaldehyde or with formaldehyde and urea, mono- or dialkylsuccinic acid ester sulfonates; and protein hydrolyzates and lignosulfite waste liquors. The aforementioned sulfonic acids are advantageously used in the form of their neutral or optionally basic salts.

The cationic surfactants include, for example, quaternized ammonium compounds, in particular alkyltrimethylammonium and dialkyldimethylammonium halides and alkyl sulfates, and also pyridine and imidazoline derivatives, in particular alkylpyridinium halides.

The nonionic surfactants include, for example:
- fatty alcohol polyoxyethylene esters, for example lauryl alcohol polyoxyethylene ether acetate,
- alkyl polyoxyethylene and polyoxypropylene ethers, e.g. of isotridecyl alcohol and fatty alcohol polyoxyethylene ethers,
- alkylaryl alcohol polyoxyethylene ethers, e.g. octylphenol polyoxyethylene ether,
- alkoxylated animal and/or vegetable fats and/or oils, for example corn oil ethoxylates, castor oil ethoxylates, tallow fatty ethoxylates,
- glycerol esters, such as, for example, glycerol monostearate,
- fatty alcohol alkoxylates and oxo alcohol alkoxylates, in particular of the type RO-(R₁₈O)ᵣ(R₁₉O)ₛR₂₀ where R₁₈ and R₁₉ independently of one another = C₂H₄, C₃H₆, C₄H₈ and R₂₀ = H or C₁-C₁₂-alkyl, R = C₃-C₃₀-alkyl or C₆-C₃₀-alkenyl, r and s independently of one another are 0 to 50, where both cannot be 0, such as isotridecyl alcohol and oleyl alcohol polyoxyethylene ether,
- alkylphenol alkoxylates, such as, for example, ethoxylated isooctyl-, octyl- or nonylphenol, tributylphenol polyoxyethylene ether,
- fatty amine alkoxylates, fatty acid amide and fatty acid diethanolamide alkoxylates, in particular their ethoxylates,
- sugar based surfactants, sorbitol esters, such as, for example, sorbitan fatty acid esters (sorbitan monooleate, sorbitan tristearate), polyoxyethylene sorbitan fatty acid esters, alkyl polyglycosides, N-alkylgluconamides,
- alkyl methyl sulfoxides,
- alkyl dimethylphosphine oxides, such as, for example, tetradecyl dimethylphosphine oxide.

In a preferred embodiment the surfactant is at least one alkoxilated fatty alcohol. In a most preferred embodiment the surfactant is at least Triton X 100, Plurafac LF 403, Emulan TO 2080, Lutensol TO 80, Pluronic P 123, Pluronic PE 7400, Pluronic 10300, Pluronic 10500, TWEEN 80, Tergitol NP9.

The weight ration of the surfactant and the lipophilic component in the aqueous dispersion can be between 0.1%:30%, preferably between 1%:20% and 5%:20%.

The concentration of the lipophilic substance in the aqueous dispersion can be between 0.5% by weight to 20% by weight, preferably 1 % by weight to 5% by based on the weight of the oil phase.

The concentration of the surfactant in the aqueous dispersion can be between 5% by weight to 30% by weight, preferably 10% by weight to 25% by weight based on the weight of the oil phase. If the lipophilic component is highly viscous, a phase inversion process can be used in which the oil phase is mixed with the surfactant and a small amount of water, forming a water-in-oil dispersion inverts to an oil-in-water dispersion when sheared. Further water can be added to dilute the dispersion.

### Step b1) or b2)

In step b1) of the process according to the invention, a water glass solution and an acid are added to the aqueous dispersion and the addition is effected in a way that the pH value of the mixture is in the range of 6 to 9.

In the first embodiment of the process of the invention, a water glass solution is added to the dispersion of step a).

In step b2) of the process according to the invention, a silicic acid solution and a base are added to the aqueous dispersion and the addition is effected in a way that the pH value of the mixture is in the range of 6 to 9.

In the second embodiment of the process of the invention, a silicic acid solution is added to the dispersion of step a).

The term "water glass" used herein is the common name for a compound comprising sodium silicate, in particular sodium or potassium metasilicate (Na₂SiO₃/K₂SiO₃), which is readily soluble in water and produces an alkaline solution. In neutral and alkaline solutions water glass is stable, but in acidic solutions the silicate ion forms silicic acid. Water glass is also characterized by the molar ration of SiO₂ and alkali oxide (for example Na₂O). Commercial water glass has a molar ration of from 1 to 4. The water glass module according the invention is preferably 2.4 to 3.4. It is of a critical importance that the process of the invention a pH is in a certain range maintained. One preferred procedure for maintaining the pH constant is the application of an in situ pH electrode coupled to dosing pumps which adjust the addition rates of the acidifying agent and the water glass, respectively. However, even more sophisticated automated reactor systems may be applied. A further possibility is to add the required acid continuously by a calculated rate based on the concentrations of water glass and acid.

The term "silicic acid" used herein are compounds which have the general formula [SiO₂ x n (H₂O)]. The technical way to produce silicic acid is to neutralize and acidifies alkalisilicate solution rapidly down to low pH, preferably ≤ 2, where the corresponding silicic acid is formed and further polycondensation to higher oligomers and polymeric sols and gels is delayed. The alkali silicate solution must be added rapidly to a strong acid, preferably H₂SO₄ or HCl, in very fine streams during rapid stirring. Metasilicate solutions or solutions of water glass solutions can be used. Another way to produce silicic acid is that metasilicate solutions or solutions of water glass solutions are run over a common ionexanger. Silica is formed by increasing the pH value.

The water glass solution employed in step b1) is preferably added as a solution containing 0.1 % by weight to 35% by weight SiO₂, preferably 1 % by weight to 30% by weight SiO₂, in particular 2% by weight to 25% by weight SiO₂ based on the total weight of the water glass solution.

The silicic acid solution employed in step b2) is preferably added as a solution containing 0.1 % by weight to 35% by weight SiO₂, preferably 1 % by weight to 30% by weight SiO₂, in particular 2% by weight to 25% by weight SiO₂ based on the total weight of the water glass solution.

The concentration of the water glass solution employed in step b1) in the aqueous dispersion can be between 35% by weight to 245% by weight, preferably 40% by weight to 185% by weight based on the weight of the oil phase.

The concentration of the silicic acid solution employed in step b2) in the aqueous dispersion can be between 35% by weight to 245% by weight, preferably 40% by weight to 185% by weight based on the weight of the oil phase.

In order to maintain the pH value of the mixture during the addition in step b1) in the intended range, preferably the water glass solution and the acid are added through two separate inlets. Preferably, a constant pH value is maintained by adjusting the addition speed of the water glass solution and/or the acid in the course of the addition period in accordance with the actual pH value of the mixture.

It has been found favourable if the acid is an inorganic acid, an aqueous solution of an inorganic acid, organic acid or an aqueous solution of an organic acid. Particular examples of the inorganic or organic acid are nitric acid, sulphuric acid, hydrochloric acid, phosphoric acid, acetic acid, formic acid, citric acid.

In order to maintain the pH value of the mixture during the addition in step b2) in the intended range, preferably the silicic acid solution and a base are added through two separate inlets. Preferably, a constant pH value is maintained by adjusting the addition speed of the silicic acid solution and/or the base in the course of the addition period in accordance with the actual pH value of the mixture.

Examples of suitable bases are alkali and alkaline-earth hydroxides, alkali and alkaline-earth carbonates and hydrogen carbonates, ammonia (NH₃), primary, secondary and tertiary amines. Preferably, the base is selected from NaOH, KOH, Mg(OH)₂, Ca(OH)₂, Na₂CO₃, K₂CO₃, CaCO₃, NaHCO₃, KHCO₃, ammonia (NH₃), tri(C₁-C₄ alkyl)amines, e.g. trimethylamine or triethylamine, and mixtures thereof.

In general, the addition of the water glass solution in step b1) or the addition of the silicic acid solution in step b2) is carried out in a range of 10 to 80°C, preferably in a range of 20 to 75°C, in particular in a range of 25 to 70°C.

According to a further development, in step b1) or b2) processing time that means the addition of water glass solution and the acid employed in step b1) or the addition of the silicic acid solution and the base employed in step b2) into the aqueous dispersion are normally added over a period of 0.5 minutes to 24 hours, preferably for a period of 8 hours to 14 hours. The period of adding depends on the batch size. For example, if the batch size is between 0.1 L to 5 L the water glass solution and acid (step b1)) or the silicic acid solution and the base (step b2)) are added over a period of 0.5 to 14 hours. The period of adding depends also on the concentration of the liquid substances. For example, if the concentration of the liquid substances is 4% by weight the water glass solution (step b1)) and acid or the silicic acid solution and the base (step b2)) are added over a period of 0.5 to 14 hours. The period of adding depends also on the concentration of the water glass solution/silicic acid solution. For example, if the concentration of the water glass solution or silicic acid solution is 2.4% by weight the adding of it and the adding of the acid needs a period of 0.5 to 14 hours. Furthermore, the dispersion is reacted at temperature in the range of 10°C to 80°C, preferably in a range of 20°C to 75°C, in particular at in a range of 25°C to 70°C. The addition is effected such that the pH of the mixture is in the range of 6 to 9, preferably 7 to 9, in particular 7.5 to 8.9.

In a preferred embodiment, the dispersion is reacted after adding the water glass solution employed in step b1) or the silicic acid solution employed in step b2) for a period of 5 minutes to 12 hours, preferably for 30 min to 4 hours. Furthermore, the dispersion is reacted at temperature in the range of 10°C to 80°C, preferably in a range of 20°C to 75°C, in particular at in a range of 25°C to 70°C. In addition to the above-mentioned moderate temperatures and ambient conditions, the method of the present invention does not require time-consuming processes, but represents a time-efficient method allowing for the production of large quantities of microcapsule composition with a silica shell.

According to a further embodiment of the present invention, the water glass solution comprises optionally at least one additive which is selected from surface modifying agents, pH regulating agents and mixtures thereof. Appropriate surface modifying agents may be chosen from ethanolamines, polyalkylene oxides, polyalkylene oxide block copolymers with polyolefines, or fatty alcohols. Alkoxy silanes, or other metal alkoxides (e.g. Mg, Ti) can be used for surface modification. Appropriate pH regulating agents are known to a person skilled in the art, but buffer systems based or acetic acid, carbonate buffer systems or hydrogenphosphate/dihydrogenphosphate buffers are preferred.

After the reaction in step b1) or b2) is complete, the microcapsule composition comprising core-shell particles is usually obtained as a dispersion in an aqueous solvent. Depending on the intended use, it is possible to employ this aqueous dispersion without a further isolation or purification step. In a preferred embodiment of the present invention, the microcapsules may be recovered from the dispersion obtained in step b1) or b2). Recovery of the microcapsule composition can be achieved by any known liquid removal technique, for example by filtering, centrifugation, spray drying, spray chilling, oven drying or lyophilisation, preferably by spray drying.

Optionally, the dispersion of microcapsules obtained in step b1) or b2) can be subjected to a further purification step prior to the isolation of the microcapsules. For example the dispersion of microcapsules obtained in step b1) or b2) can be subjected to a cross-flow-filtration. The cross-flow-filtration can be carried out in a customary manner. As a rule, a cross-flow is generated on the membrane surface at a speed of about 2.5 to 3 m/s. In general, the membrane consists of ceramic and has a pore size of about 20 to 40 nm. The dissolved salts are separated from the solution. As a rule, the electrical conductivity of the solution is about 30 microSiemens after the cross-flow-filtration step. In another embodiment example, the dispersion of microcapsules obtained in step b1) or b2) can be subjected to a dialysis. The dialysis can be carried out in a customary manner.

The spray drying of the dispersion of microcapsules can be carried out in a customary manner. In general, the inlet temperature of the hot stream is in the range from 100 to 200°C, preferably 120 to 160°C and the outlet temperature of the air stream is in the range from 30 to 90°C, preferably from 60 to 80°C. The spraying of the microcapsule dispersion in the hot air stream can be carried out, for example, by means of single-fluid or multifluid nozzles or by means of a rotating disk. The microcapsule composition is normally separated off by using cyclones or filters. The sprayed microcapsule dispersion and the hot air stream are preferably conveyed in concurrent.

In another embodiment, drying of the dispersion of microcapsules can be carried out at a temperature in the range from 20 to 100°C for a period of 30 min to 15 h, preferably drying of the dispersion of microcapsules can be carried out at a temperature in the range from 20 to 100°C for a period of 30 min to 15 h in combination with reduced pressure.

A further advantage is to be seen in that the aqueous microcapsule compositions according to the invention can as a rule be dried to a redispersible powder. That is, by removal of the aqueous phase during the drying, a finely divided powder is obtained which can, without any bother, be redispersed in water without the occurrence of a significant change in particle size.

A further aspect of the present invention is a microcapsule composition obtainable by the method described above.

Preferably, the particles of the microcapsule composition according to the invention have an average particle size in the range of 50 to 50000 nm, more preferably of 100 to 3000 nm, in particular of 150 to 500 nm. The particle size can be determined by known methods, e.g. transmission electron microscopy (TEM) analysis, scanning electron microscopy (SEM), atom force microscopy (AFM) or by light scattering.

Preferably, the particles in the microcapsule composition according to the invention have a shell with thickness of 1 to 50 nm, more preferably 5 to 20 nm.

A further aspect of the present invention is the use of a microcapsule composition as defined above or obtainable by the above-mentioned process in a polymer composition, cosmetic composition, pharmaceutical composition, home care product, an adhesive or coatings.

A further aspect of the present invention is the use of the microcapsule composition as defined above or obtainable by the above-mentioned process for the delivery of an active substance, preferably selected from flame retardants, UV-stabilizers, UV-protecting agents, fragrances, flavors, vitamins, aromatic compounds, cosmetic or therapeutic agents, anti fouling agents.

A further aspect of the present invention is the use of the microcapsule composition as defined above or obtainable by the above-mentioned process for the encapsulation of a latent heat storage material.

The microcapsule composition of the present invention is particularly useful for the encapsulation of at least one flame retardant, in particular for use in a polymer composition. Advantageously, the thus encapsulated flame retardant is only released in case of need, i.e. if the article containing the microcapsule composition is exposed to fire or excessive heat.

The microcapsule composition of the present invention can be incorporated into various polymer matrices. The incorporation of the microcapsule composition and optional further components into a polymer composition can be carried out by known methods, such as dry mixing in the form of a powder, or wet mixing in the form of dispersions. The microcapsule composition and optional further components may be incorporated, for example, before or after molding or also by applying the microcapsule composition and optional further components to the polymer composition, with or without subsequent evaporation of the solvent. The microcapsule composition and optional further additives may be added direct into the processing apparatus (e.g. extruders, internal mixers, etc.), e.g. as a dry mixture or dispersion. The incorporation can be carried out e.g. in any heatable container equipped with a stirrer, e.g. in a closed apparatus, such as a kneader, mixer or stirred vessel. Examples of processing of the compositions comprising at least one polymer and a microcapsule composition according to the invention are: extrusion, blow molding, injection blow molding, extrusion blow molding, rotomolding, in mold decoration (back injection), slush molding, injection molding, co-injection molding, forming, compression molding, pressing, film extrusion (cast film; blown film), fiber spinning (woven, non-woven), drawing (uniaxial, biaxial), annealing, deep drawing, calandering, mechanical transformation, sintering, coextrusion. The incorporation is preferably carried out in an extruder or in a kneader according to methods known in literature.

The microcapsule composition of the present invention is also particularly useful as a latent storage material. The microcapsule composition of the present invention can be incorporated into binding building materials containing minerals, siliceous or polymeric binders. The microcapsule composition of the present invention can also be incorporated and/or coated on textiles.

The microcapsule compositions according to the present invention inhibit diffusion or leaching of the core which comprises of lipophilic components from the microcapsule. When encapsulating the lipophilic components, it is preferred that the rate of the diffusion or leaching is as low as possible. The decapsulating of the lipophilic components can be achieved by changing the pH value to a value of at last 13. For example, the use of ethanol will dissolve the lipophilic component due to the porous SiO₂ shell.

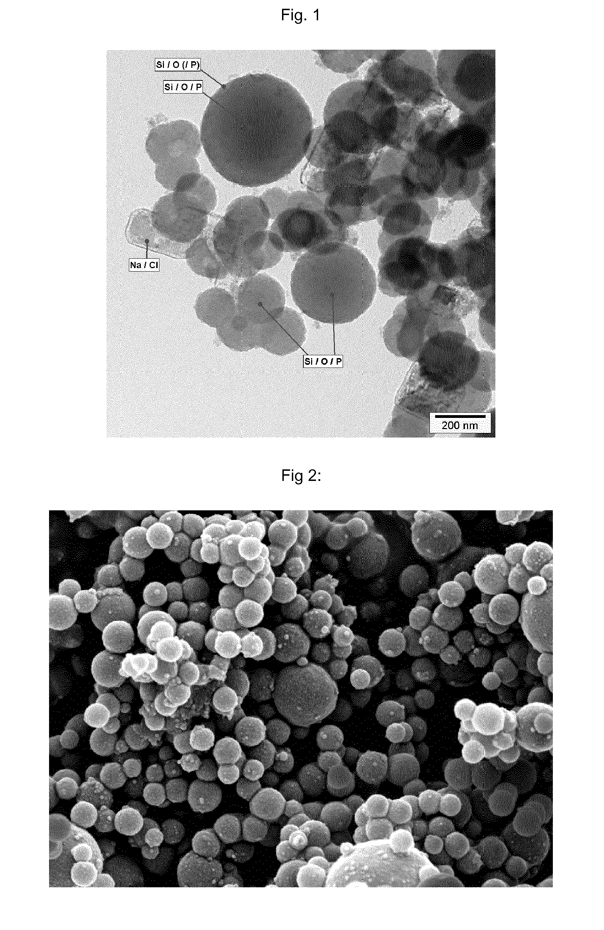

- Figure 1:: TEM analysis (Transmission electron microscopy), encapsulation of resorcinol bis(diphenyl phosphate) (RDP)
- Figure 2:: SEM analysis (Scanning electron microscopy), spherical particles of encapsulated resorcinol bis(diphenyl phosphate) (RDP)

The following examples are intended to further illustrate the present invention without limiting its scope in any way.

### Table 1: Fire Test

### I. Apparatuses

The particle sizes given here are weight-average particle sizes, and they can be determined by dynamic light scattering, e.g. with a Mictrotrac Nanotrac 250.

Ultrasonic: Hielscher; UP200S; 40 mm Sonotrode; Cycle 0.5; Amplitude 100% TEM-Analysis: Philips (FEI) CM120 TEM
Mini-Compounder: DSM Midiextruder and Injection molder

### II. Ingredients

Triton X^{®} 100: (Octylphenol Ethoxylate) by Dow Chemical Company
Pluronic^{®} PE 10300, BASF
Resorcinol bis(diphenyl phosphate) (Fyrolflex RDP, ICL industrial products)
Tergito^{®}NP9 by Dow Chemical Company
Water glass solution, BASF SE

### III. Preparation

### Example 1:

### (general procedure for the preparation of the flame retardants of example 5)

In a tempered vessel 6 g of Triton X 100 (corresponding to 20% of the amount of the lipophilic component) were dissolved in 714 g of demineralised water. 30 g resorcinol bis(diphenyl phosphate) (RDP) (4% based on the total weight) were added while stirring. This mixture was dispersed by ultrasonic treatment for 30 min. 375 mL of a 2.36% water glass solution were added drop-wise to the tempered vessel at 60°C. During the addition of the water glass solution the pH in the tempered vessel was kept constant at a value of 8 by addition of the corresponding amount of 1 M HCl. After reacting 12.5 h, the resulting suspension was filtered though a glass frit (0.45 µm filter) and washed several times with demineralised water. The product was dried over night (8 h) at 25°C under reduced pressure.

### Example 2:

In a tempered vessel 6 g of Triton X 100 (corresponding to 20% of the amount of the lipophilic component) were dissolved in 714 g of demineralised water. 30 g resorcinol bis(diphenyl phosphate) (RDP) (4% based on the total weight) were added while stirring. This mixture was dispersed by ultrasonic treatment for 30 min. 375 mL of a 2.36% water glass solution were added drop-wise to the tempered vessel at 60°C. During the addition of the water glass solution the pH in the tempered vessel was kept constant at a value of 8 by addition of the corresponding amount of 1 M HNO₃. After reacting 12.5 h, the resulting suspension was filtered though a glass frit (0.45 µm filter) and washed several times with demineralised water. The product was dried over night (8 h) at 25°C under reduced pressure.

### Example 3:

In a tempered vessel 0.3 g of Pluronic PE 10300 (corresponding to 5% of the amount of the lipophilic component) were dissolved in 48 g of demineralised water. 6 g Decan were added while stirring. This mixture was dispersed by ultrasonic treatment for 30 min. 90 mL of a 2.36% water glass solution were added drop-wise to the tempered vessel at 60°C. During the addition of the water glass solution the pH in the tempered vessel was kept constant at a value of 8 by addition of the corresponding amount of 1 M HCl. After reacting 12.5 h, the resulting suspension was filtered though a glass frit (0.45 µm filter) and washed several times with demineralised water. The product was dried over night (8 h) at 25°C under reduced pressure.

### Example 4:

In a tempered vessel 0.3 g of Tergitol NP9 (corresponding to 20% of the amount of the lipophilic component) were dissolved in 97.6 g of demineralised water. 6 g resorcinol bis(diphenyl phosphate) (RDP) were added while stirring. This mixture was dispersed by ultrasonic treatment for 30 min. 15 mL of a 2.36% water glass solution were added drop-wise to the tempered vessel at 60°C. During the addition of the water glass solution the pH was kept constant at a value of 8 by addition of the corresponding amount of 1 M HNO₃. After reacting 12.5 h, the resulting suspension was filtered though a glass frit (0.45 µm filter) and washed several times with demineralised water. The product was dried over night (8 h) at 25°C under reduced pressure.

### Example 5: Efficiency of the flame retardant test according to DIN IEC 60695-11-10

A stable dispersion made of resorcinol bis(diphenyl phosphate) (RDP) (2% in water) and Triton X 100 (0.4%) is prepared using the general procedure according to example 1 and dried as described to obtain a white powder.

The obtained material was tested in polybutylene terephthalate PBT, Ultradur® B4300 G6 from BASF SE) for its activity as flame retardant additive. 22.7% of the microencapsulated flame retardant and 15% of a known flame retardant synergist (synergist 1 = melamine cyanurate, Melapur® MC from BASF SE, and synergist 2 = melamine polyphosphate, Melapur® 200 from BASF SE,) were incorporated (compounded) into glass fibre reinforced polymer. The fire test was performed by the method UL 94 V ("Tests for Flammability of Plastic Materials for Parts in Devices and Applications", corresponding to IEC/DIN EN 60695-11-10 und -20, thickness of the test specimen: 1.6 mm). Table 1 demonstrates that the encapsulated flame retarded resorcinol bis(diphenyl phosphate) (RDP) achieves the highest fire protection class V0.

**Table 1: Fire Test**

| Composition | 1 | 2 |
|---|---|---|
| | [wt.-%] | [wt.-%] |
| PBT | 62.3 | 62.3 |
| encapsulated flame retardant additive | 22.7 | 22.7 |
| Synergist 1 | 7.5 | - |
| Synergist 2 | 7.5 | 15 |
| Burninq test | 7 x V0, 3 x V1 | 3 x V0, 2 xV- |

## Claims

1. A process for the preparation of a microcapsule composition, wherein the shell of the microcapsules is essentially made of silica and the core comprises at least one lipophilic component, comprising the steps of:
a) providing an aqueous dispersion comprising at least one lipophilic component (A) and
b1) adding to the aqueous dispersion provided in step a) a water glass solution and an acid, or
b2) adding to the aqueous dispersion provided in step a) a silicic acid solution and a base,
wherein the addition is effected such that the pH of the mixture resulting during the addition of the water glass solution in step b1) or during the addition of silicic acid solution in step b2) is kept in a range of 6 to 9.

2. A process according to claim 1, wherein the aqueous dispersion provided in step a) additionally comprises at least one surfactant.

3. A process according to claim 1 or 2, wherein the lipophilic component (A) is liquid under the conditions of step b1) or b2).

4. A process according any of the preceding claims, wherein the lipophilic component (A) has a solubility in water at 23°C and 1013 mbar of ≤ 50 mg/mL, preferably ≤ 5 mg/mL, in particular ≤ 1 mg/mL.

5. A process according to any of the preceding claims, wherein the lipophilic component (A) is selected from flame retardants, UV-stabilizers, UV-protecting agents, fragrances, flavors, vitamins, aromatic compounds, cosmetic agents, therapeutic agents or anti fouling agents.

6. A process according to any of the preceding claims, wherein the lipophilic component (A) comprises or consists of a flame retardant, in particular selected from halogen-containing flame retardants, nitrogen-based flame retardants, organophosphorus compounds or mixtures thereof.

7. A process according to claim 6, wherein the flame retardant is selected from pentabromodiphenyl ether, octabromodiphenyl ether, decabromodiphenyl ether, tetrabromobisphenol A, hexabromocyclododecane, chlorendic acid, clorinated paraffins, melamine, urea, tris(2-chloroethyl) phosphate, tris(1-chloro-2-propyl) phosphate, tris(1,3-dichloro-2-propyl) phosphate, triphenyl phosphate, triethyl phosphate, isodecyl diphenyl phosphate, tris(2-ethylhexyl) phosphate, tri-n-butyl phosphate, tri-isobutyl phosphate, tricresyl phosphate, isopropylated triphenyl phosphate with different isopropyl rates, bisphenol-A-bis(diphenyl phosphate), resorcinol bis(diphenyl phosphate) and mixtures thereof.

8. A process according to any of the preceding claims, wherein during the addition of the water glass solution in step b1) or during the addition of the silicic acid solution in step b2) the pH-value of the mixture is kept in a range of from 7 to 9, preferably in a range from 7.5 to 8.9.

9. A process according to any of the preceding claims, wherein during the addition of the water glass solution in step b1) or during the addition of the silicic acid solution in step b2) the temperature of the mixture is kept in a range of from 10°C to 80°C, preferably from 20 to 75°C, in particularly from 25 to 70°C.

10. A process according to any of the preceding claims, wherein in step b1) the water glass solution or in step b2) the silicic acid solution contains at least 0.1 to 35% by weight, preferably at least 1 to 30% by weight, in particularly at least 2 to 25% by weight SiO₂, based on the total weight of the water glass solution.

11. A process according to any of the preceding claims, wherein the addition in step b1) or b2) is effected in a time of from 1 to 72 hours, preferably from 5 to 48 hours.

12. A process according to any of the preceding claims, wherein the dispersion provided in step a) comprises at least one additive different from (A), preferably selected from surface modifying agents, pH-regulating agents and mixtures thereof.

13. A process according to any of claims 2 to 12, wherein the surfactant comprises at least one nonionic surfactant, in particular at least one alkoxilated fatty alcohol.

14. A process according to any of the preceding claims, wherein the microcapsule composition obtained in step b1) or b2) is subjected to at least one further process step, preferably selected from separation, purification and drying steps and a combination of them.

15. A process according to any of the preceding claims, wherein the microcapsule composition obtained in step b1) or b2) is subjected to a drying.

16. A microcapsule composition, obtainable by a process as defined in any one of the claims 1 to 15.

17. A microcapsule composition according to claim 16, wherein the particles having an average particle size in the range from 50 to 50000 nm, preferably from 100 to 3000 nm, more preferably from 150 to 500 nm.

18. A microcapsule composition according to claim 16 or 17, wherein the shell having a thickness of 1 to 50 nm, preferably 5 to 20 nm.

19. The use of a microcapsule composition as defined in any of claims 16 to 18 or obtainable by a process according to any of claims 1 to 15 in a polymer composition, cosmetic composition, pharmaceutical composition, home care product, an adhesive and coatings.

20. The use of the microcapsule composition as defined in any of claims 16 to 18 or obtainable by a process according to any of claims 1 to 15 for the delivery and release of an active substance from microcapsules, preferably selected from flame retardants, UV-stabilizers, UV-protecting agents, fragrances, flavours, vitamins, aromatic compounds, cosmetic or therapeutic agents, anti fouling agents.

21. The use of the microcapsule composition as defined in any of claims 16 to 18 or obtainable by a process according to any of claims 1 to 15 for the encapsulation of a latent heat storage material.
